# EUROPEAN PATENT APPLICATION

(11) **EP 1 964 629 A2**
(43) Date of publication of application: **03.09.2008**
(21) Application number: 08250481.2
(22) Date of filing: 08.02.2008
(51) Int. Cl.: B22F 1/00

(54) **Porous metal foam structures**

(30) Priority: 21.02.2007 US 677140
(71) Applicant: DePuy Products, Inc., Warsaw, IN 46581 (US)
(72) Inventor: Liu, Hengda, Warsaw, IN 46580 (US)
(74) Representative: Belcher, Simon James

(57) **Abstract**

A method of making porous metal foam structures includes the steps of combining a liquid-extractable, pore-forming agent with a metal powder in the presence of a liquid in which the pore-forming agent is soluble, thereby forming a mixture, compacting the mixture to form a green body, and dissolving the pore-forming agent from the green body to produce a metal skeleton.

## Description

The present invention relates to porous metal foam structures and processes for making such structures.

Porous metal foam structures have a number of uses, including as medical implants. Porosity in such structures can be achieved by mixing the metal as a powder with a pore-forming agent (PFA) and then pressing the mixture into the desired shape to form a green body. After the PFA is removed, the metal skeleton can be sintered to achieve the desired properties for the porous metal foam structure.

One way of removing the PFA from the green body is to "burn out" the PFA. This can lead to a variety of potential problems, such as contamination of the furnace, formation of undesirable metal compounds in the porous structure induced by the reaction between the metal and PFA, and usage of relatively large amounts of energy. There are also certain storage problems associated with green bodies produced in this manner, including noxious odor formation.

In one aspect, the present invention provides processes that comprise combining a liquid-extractable, pore-forming agent with a metal powder in the presence of a liquid in which the pore-forming agent is soluble, thereby forming a mixture that is compacted to form a green body. The pore-forming agent is then dissolved from the green body to produce a metal skeleton that can be sintered to form a sintered metal foam structure such as a porous metal implant. The present invention also provides porous metal implants and other sintered foam structured produced by such methods.

In another aspect, the invention provides a metal implant having at least one of:
a flexural yield strength of at least 90 MPa, and
a compressive yield strength of at least 65 MPa,
in which the implant has been formed from a mixture of a liquid extractable pore forming agent and a metal powder, and having at least 65% porosity.

The present invention provides processes that involve combining a liquid-extractable pore-forming agent (PFA) with a metal powder in the presence of a liquid in which the PFA is soluble. It is understood that possible PFA/liquids combinations include PFAs that are soluble in organic liquids paired with an organic liquid, or PFAs that are soluble in non-organic liquids paired with a non-organic liquid.

In certain embodiments, the liquid is aqueous. Preferably, the liquid includes at least about 75 wt-% water, more preferably at least about 90 wt-% water, even more preferably at least about 95 wt-% water. Representative liquids include water (such as reverse osmosis water, deionized water, distilled water, and/or deoxygenated water) or an aqueous carbohydrate solution.

Although the amount of liquid used will depend upon the nature of the metal powder and PFA and the processing conditions employed, it has been found that the use of about 450 1 to about 1050 1 per 100 cm³ of the pre-compaction mixture should be used, more preferably about 600 1 to about 750 1 per 100cm³ of pre-compaction mixture.

PFAs according to the present invention are particulate materials that are soluble in a fluid of interest. Representative PFAs include sodium chloride, ammonium chloride, calcium chloride, magnesium chloride, aluminum chloride, potassium chloride, nickel chloride, zinc chloride, ammonium bicarbonate, sodium hydrogen phosphate, sodium dihydrogen phosphate, potassium dihydrogen phosphate, potassium hydrogen phosphate, potassium hydrogen phosphite, potassium phosphate, magnesium sulfate, potassium sulphate, alkaline earth metal halides, crystalline carbohydrates (including sucrose and lactose classified as monosaccharides, disaccharides, and trisaccharides), polyvinyl alcohol (PVA), polyethylene oxide, a polypropylene wax (such those available from Micro Powders, Inc., Tarrytown, NY, under the PROPYLTEX trademark), sodium carboxymethyl cellulose (SCMC), polyethyleglycol-polypropylene-polyethyleneglycol copolymers (PEG-PPG-PEG, such as those available from BASF, Ludwigshafen, Germany under the PLURONIC trademark), and combinations thereof.

The PFA can be present in a wide variety of particle sizes and particle size distributions suitable to produce a pore size and pore size distribution. Certain preferred particle size ranges are from about 200 µm to about 600 µm, from about 200 µm to about 350 µm, and from about 350 µm to about 550 µm.

Virtually any type of metal powder known in the field of powder metallurgy can be used in the methods of the present invention. Preferred metal powders are those that are formed from titanium, cobalt, chromium, nickel, magnesium, tantalum, niobium, zirconium, aluminum, copper, molybdenum, tungsten, stainless steel, or alloys thereof (e.g., Co-Cr alloy). In one embodiment, the metal powder is titanium or a titanium alloy such as Ti-6Al-4V.

The metal powder also can be present in a wide variety of particle sizes and particle size distributions. Certain preferred particle size ranges are from about 20 µm to about 100 µm, from about 25 µm to about 50 µm, and from about 50 µm to about 80 µm.

Those skilled in the art will recognize that the proportions of metal powder and PFA will vary depending upon the type of structure sought to be produced. In certain embodiments of the present invention, the ratio of metal powder to PFA is about 40:60 to about 10:90, preferably about 25:75 with the PFA.

After the metal powder, PFA, and liquid are mixed, the resulting mixture is compacted to form a green body. The compacting step can be carried out via any of the many techniques known in the art, including uniaxial die and punch, biaxial die and punch, or cold or rubber isostatic press. Preferably, the compacting pressure is at least about from about 138 MPa (20 ksi), more preferably at least about 207 MPa (30 ksi). Preferably, the compacting pressure is not more than about 414 MPa (60 ksi), more preferably not more than about 310 MPa (45 ksi). Once formed, the green body may be machined by known techniques such as cutting, milling, turning, drilling, and/or facing.

The PFA can be removed from the green body using any liquid capable of dissolving the PFA, thus revealing the metal skeleton. As with the liquid that is mixed with the metal powder and PFA prior to compaction, the dissolving liquid is preferably aqueous, and more preferably water (such as reverse osmosis water, deionized water, distilled water, and/or deoxygenated water) or an aqueous carbohydrate solution. The liquid that is used to dissolve the PFA can be the same as or different from the liquid that is mixed with the metal powder and PFA prior to compaction, e.g., the chemical identity of the components in the respective liquids and/or their relative proportions can be the same or different.

The dissolution step can be effected by, for example, immersing the green body in a bath containing a liquid in which it is soluble or contacting the green body with a stream of that liquid. The temperature range for the liquid used in the dissolution step is above its freezing point but below its boiling point, and preferably about 10°C to about 80°C (about 50°F to about 176°F). Certain steps known to affect dissolution may be implemented, for example, the bath solution can be circulated or portions of the bath solution periodically replaced with fresh solution.

The metal skeleton obtained upon removal of the PFA may also be machined, such as by cutting, milling, turning, drilling, and/or facing the skeleton.

The metal skeleton typically will be sintered to impart the desired properties. While all suitable sintering conditions are contemplated, sintering for titanium or Ti-6Al-4V alloy typically will be performed at temperatures of from about 1149°C (2100°F) to about 1482°C (2700°F) (especially about 1371 °C(2500°F)) and/or for about 2 hr to about 10 hr (preferably about 3 hr to about 6 hr).

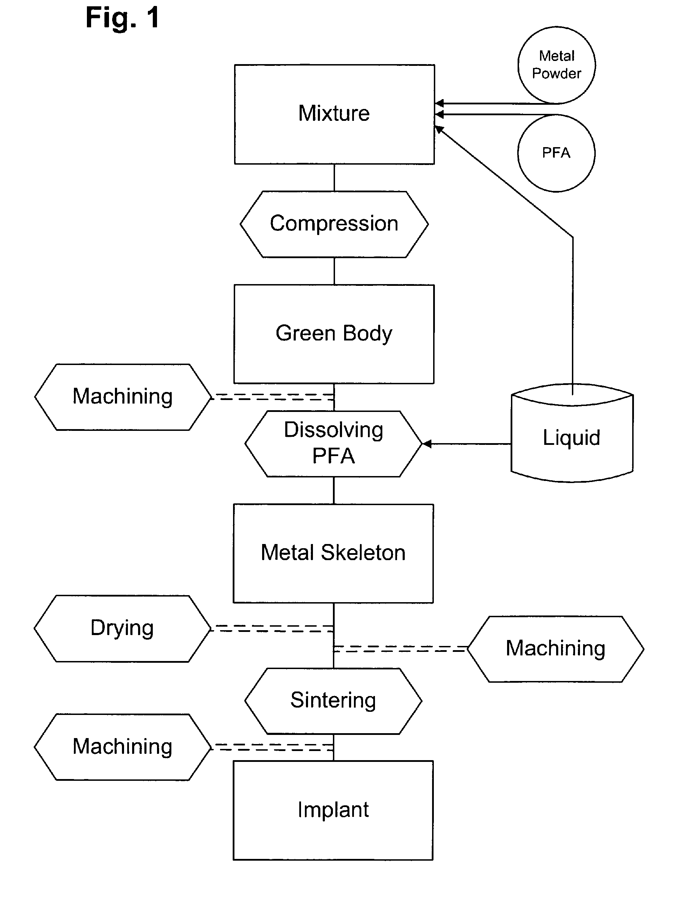

The methods of the invention can be used, for example, to produce metal implants that include a porous surface. Referring now to Fig. 1, certain processes for making such implants are depicted. Metal powder, PFA, and a liquid in which the PFA is soluble are combined to form a mixture. The mixture is compressed (for example via uniaxial, multi-axial, or isostatic compaction) in a shaped mold to form a green body. The mold determines the shape of the implant, and thus should generally be of a desired shape to avoid or at least minimize the need for substantial machining. The PFA is dissolved from the green body through contact with a liquid in which the PFA is soluble to form a metal skeleton. Optionally, the metal skeleton may be machined and/or dried to remove residual liquid. The metal skeleton is sintered, and afterwards optionally machined to form a porous metal implant. Those skilled in the art are aware of suitable shapes for such implants and the properties that they should possess, for example suitable compressive yield strength. Although the first liquid and second liquid are depicted as coming from the same source in Fig. 1, it is understood that the liquids need not be identical, only that they each be a liquid in which the pore-forming agent is soluble.

The surface of the porous metal implant may be roughened. Methods of roughening include at least one of grit blasting, etching, or plasma sputtering and are known in the art. A preferred method of etching is disclosed in US-A- 2004/0167633. A preferred method of grit blasting uses a water soluble grit, such as NaCl, to blast against the implant, thus allowing for removal of impacted grit from the pores by dissolution in an aqueous liquid.

In certain embodiments, the present invention provides metal implants or other types of metal skeletons having a porosity of from about 60% to about 85 % (preferably about 65% to about 75%) as measured by volume, the forced intrusion of liquid mercury, and cross-section image analysis. It is understood that the porosity can be a product of metal to PFA ratio, PFA size, or a combination thereof.

In one embodiment, preferred pure titanium skeletons are those that have a tensile strength of at least about 35 MPa (as measured by the standard tension testing -ASTM E8-99), or a flexural yield strength of at least about 90 MPa (as measured by three-point bend testing - ASTM E290-97a), and/ or a compressive yield strength of at least about 65 MPa (as measured by monotonic compression testing - ASTM E9-89a) at a porosity of about 65%. Particularly preferred pure titanium skeletons are those that have a tensile strength of at least about 40 MPa (measured using ASTM E8-99), or with a flexural yield strength of at least about 110 MPa (measured using ASTM E290-97a), and/ or with a compressive yield strength of at least about 75 MPa (measured using ASTM E9-89a) at a porosity of about 65%.

It is understood that titanium alloys can be used to obtain greater strengths. Preferred titanium alloy skeletons are those that have a tensile strength of at least about 60 MPa (measured using ASTM E8-99), or with a flexural yield strength of at least about 120 MPa (measured using ASTM E290-97a), and/ or with a compressive yield strength of at least about 90 MPa (measured using ASTM E9-89a) at a porosity of about 65%. Particularly preferred titanium alloy skeletons are those that have a tensile strength of at least about 90 MPa (measured using ASTM E8-99), or with a flexural yield strength of at least about 180 MPa (measured using ASTM E290-97a), and/ or with a compressive yield strength of at least about 110 MPa (measured using ASTM E9-89a) at a porosity of about 65%.

It is believed that porosity, metal powder particle size, and sintering temperature are important factors contributing to the strength of the resulting structure.

Embodiments of the invention are described by way of example with refeerence to the accompanying drawings, in which:
Fig. 1 is a schematic of a process of making a porous metal implant according to one embodiment of the present invention.
Fig. 2 is an image of a sintered metal foam structure.
Fig. 3 is an image of the sintered metal foam structure of Fig. 2 in a side view.
Fig. 4 is an image of the sintered metal foam structure of Fig. 2 in a detail view.
Fig. 5 is an optical microscope image of the sintered metal foam structure of Fig. 2.
Fig. 6 is a scanning electron microscope (SEM) image of a sintered metal foam structure at 200× magnification.
Fig. 7 is a SEM image of the sintered metal foam structure at 700× magnification.

### EXAMPLE 1

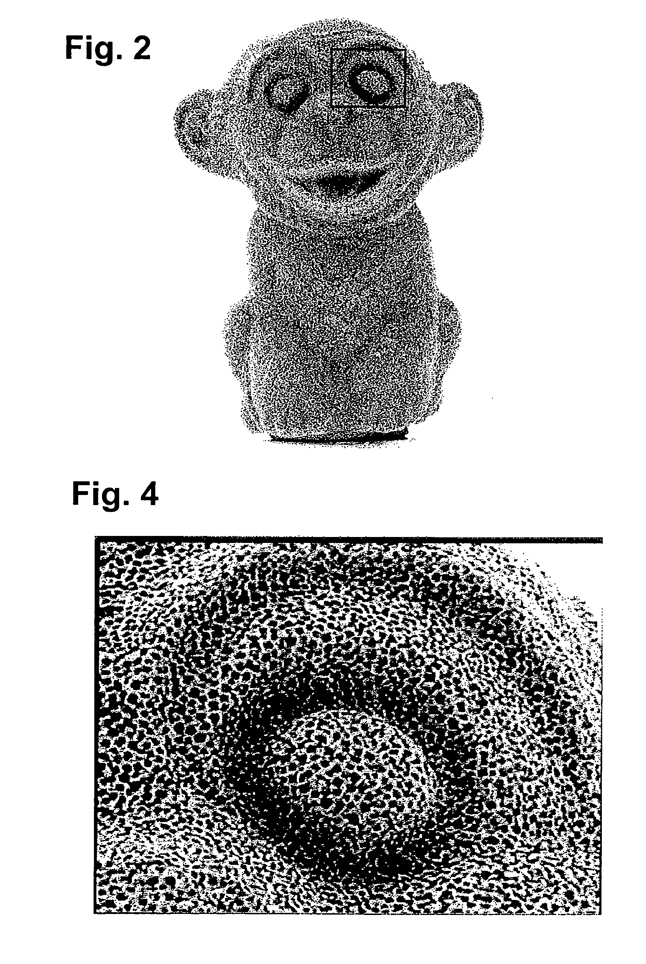

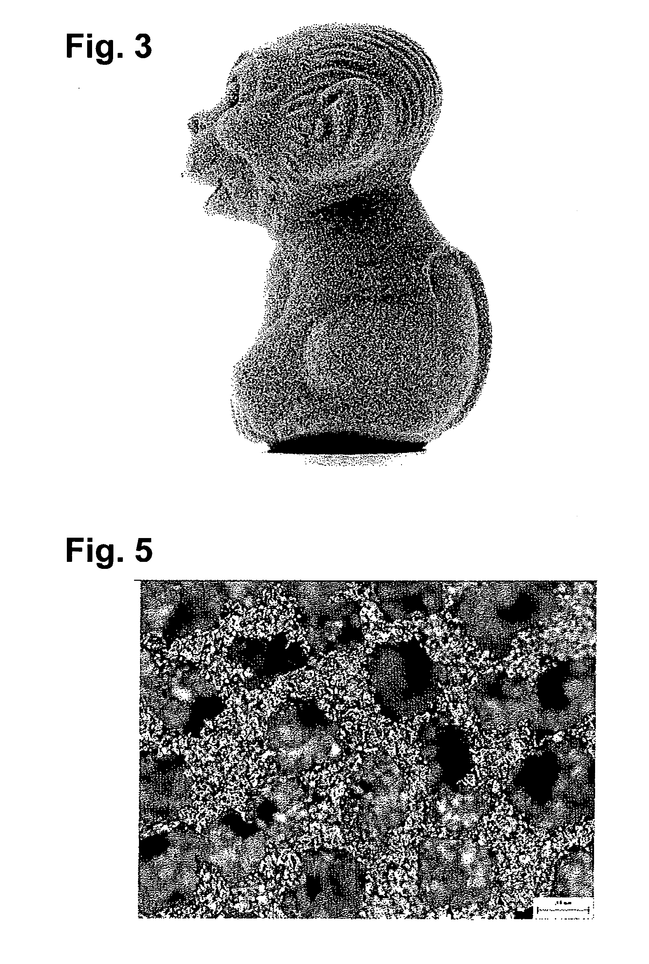

Commercial pure titanium powder (Phelly Materials, Inc. Bergenfield, NJ, USA) particle size: 45 to 75 µm and NaCl (Fisher Scientific International Inc. Hampton, NH, USA) particle size: 250 to 425 µm, as a PFA, were mixed in a ratio of approximately 25:75 Ti:PFA by volume. Reverse osmosis water was added in an amount corresponding to about 700 1 per 100 cm³ of Ti:PFA mixture. The mixture was added to a mold and compressed into a green body at a compaction pressure of 152 MPa (22 ksi). The green body was placed in a water bath until the NaCl dissolved. The resulting metal skeleton was dried at 65°C for 4 hours, and then sintered at 1204°C for 2 hours. The sintered metal foam structure is depicted in Figs. 2 to 5, which show a highly porous metal foam structure in a complex shape.

### EXAMPLE 2

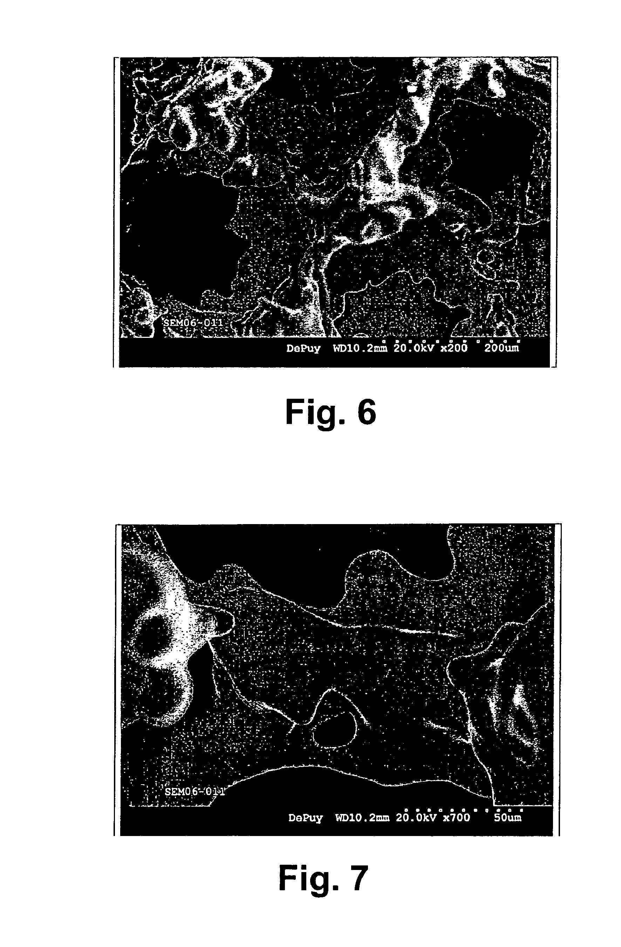

Commercial pure titanium powder particle size: 45 to 75 µm and NaCl particle size: 250 to 425 µm, as a PFA, were mixed in a ratio of approximately 20:80 Ti:PFA by volume. Reverse osmosis water was added in an amount corresponding to about 700 1 per 100 cm³ of Ti:PFA mixture. The mixture was added to a mold and compressed into a green body at a compaction pressure of 163 MPa (23.6 ksi). The green body was placed in a water bath until the NaCl dissolved. The resulting metal skeleton was first dried in the oven as in Example 1 and then sintered at 1371 °C for 3 hours. The sintered metal foam structure is depicted in Figs. 6 to 7.

### EXAMPLE 3

Titanium powder (32 to 45 µm (500 to 350 mesh)) and NaCl (425 to 500 µm) were mixed in a ratio of approximately 25:75 Ti:PFA by volume. Reverse osmosis water was added in an amount corresponding to about 700 1 per 100 cm³ of Ti:PFA mixture. The mixture was added to a mold and compressed into a green body at a compaction pressure of 207 MPa (30 ksi). The green body was placed in a water bath for about 12 hours to allow the PFA to dissolve. The resulting metal skeleton was sintered at 1731 °C for 6 hours. The sintered metal foam structures had about 65% porosity. The compressive yield strength and flexural yield strength were 82 MPa and 180 MPa, respectively, determined by performing the standard compression test and three-point bend test following ASTM E9-89a and ASTM E290-97a.

### EXAMPLE 4

Titanium powder (32 to 45 µm (500 to 350 mesh)) and NaCl (250 to 300 µm) were mixed in a ratio of approximately 25:75 Ti:PFA by volume. Reverse osmosis water was added in an amount corresponding to about 700 1 per 100 cm³ of Ti:PFA mixture. The mixture was added to a mold and compressed into a green body at a compaction pressure of 310 MPa (45 ksi). The green body was placed in a water bath for about 12 hours to allow the PFA to dissolve. The resulting metal skeleton was sintered at 1371 °C for 6 hours. The sintered metal foam structures had about 65% porosity. The compressive yield strength and flexural yield strength were 77 MPa and 196 MPa, respectively, determined as described above with reference to Example 3.

## Claims

1. A process comprising the steps of:
combining a liquid-extractable, pore-forming agent with a metal powder, and a first liquid in which the pore-forming agent is soluble, thereby forming a mixture;
compacting the mixture to form a green body; and
dissolving the pore-forming agent in a second liquid in which the pore-forming agent is soluble, thereby producing a metal skeleton.

2. A process as claimed in claim 1, in which the first liquid is aqueous.

3. A process as claimed in claim 1, in which about 450 1 to about 1050 1, preferably about 600 1 to about 750 1, of the first liquid is mixed with the pore-forming agent and the metal powder per each 100 cm³ of the mixture.

4. A process as claimed in claim 1, in which the first liquid is reverse osmosis water, deionized water, distilled water, deoxygenated water, demineralized water, or an aqueous carbohydrate solution.

5. A process as claimed in claim 1, in which the first liquid is at least about 75 wt-% water.

6. A process as claimed in claim 1, in which the pore-forming agent is sodium chloride, ammonium chloride, calcium chloride, magnesium chloride, aluminum chloride, potassium chloride, nickel chloride, zinc chloride, ammonium bicarbonate, sodium hydrogen phosphate , sodium dihydrogen phosphate, potassium dihydrogen phosphate, potassium hydrogen phosphate, potassium hydrogen phosphite, potassium phosphate, magnesium sulphate, potassium sulfate, an alkaline earth metal halide, a crystalline carbohydrate, polyvinyl alcohol (PVA), polyethylene oxide, polypropylene wax , sodium carboxymethyl cellulose (SCMC), polyethyleglycol-polypropylene-polyethyleneglycol copolymer (PEG-PPG-PEG), or a combination thereof.

7. A process as claimed in claim 1, in which the pore-forming agent has a particle size of about 200 µm to about 600 µm, preferably about 200 µm to about 350 µm, especially about 350 µm to about 550 µm.

8. A process as claimed in claim 1, in which the metal powder is formed from titanium, cobalt, chromium, nickel, magnesium, tantalum, niobium, zirconium, aluminum, copper, molybdenum, tungsten, stainless steel, or an alloy thereof.

9. A process as claimed in claim 1, in which the metal powder has a particle size of about 20 µm to about 100 µm, preferably about 25 µm to about 50 µm, especially about 50 µm to about 80 µm.

10. A process as claimed in claim 1, in which the first liquid and the second liquid are the same.

11. A process as claimed in claim 1, in which the first liquid and the second liquid are different.

12. A process as claimed in claim 1, in which the second liquid is aqueous.

13. A process as claimed in claim 1, in which the metal powder is in a ratio of volume about 40:60 to about 10:90, preferably about 25:75, with the pore forming agent.

14. A process as claimed in claim 1, which includes sintering the metal skeleton to form a porous metal implant, in which the sintering temperature is in a range from about 1149°C (2100°F) to about 1482°C (2700°F).
